# EUROPEAN PATENT APPLICATION

(11) **EP 1 338 265 A1**
(43) Date of publication of application: **27.08.2003**
(21) Application number: 03003818.6
(22) Date of filing: 20.02.2003
(51) Int. Cl.: A61K 7/06

(54) **Hair regenerator composition**

(30) Priority: 21.02.2002 ES 200200416
(71) Applicant: Adnan Ammura Taha, 08916 Badalona (Barcelona) (ES)
(72) Inventor: Adnan Ammura Taha, 08916 Badalona (Barcelona) (ES)
(74) Representative: Isern Jara, Nuria

(57) **Abstract**

Hair regenerator composition, prepared as a lotion, which contains a series of known products and product normally used for maintain and improve the appearance of the hair, it contains some differing active ingredients, namely spironolactone and flutamide. These substances can be used on their own or the two of them mixed together and used in concentrations with sufficient quantities to produce a reduction in the levels of the androgens that cause alopecia, thus, increasing the relative relationship of oestrogen.

The hair regenerator composition is used to prevent and combat hair loss.

## Description

The present invention refers to a product, presented as a lotion, to be used to prevent and combat hair from falling out.

There are numerous compositions known for the treatment of alopecia, normally presented as lotions. However, normally these products give mediocre results and do not achieve the aim sought very well.

Other products can give better results if their composition includes oestrogen (female hormones and/or parahormones). However, these products carry the danger of causing an imbalance to the human metabolism, not only as a result of their strong endocrine action, due to their being very active substances that are absorbed very easily (orally, other than through the gastro-intestinal tract or through the skin).

The hair regenerator composition that is the aim of this invention resolves and reduces to the maximum the problems and disadvantages stated above. This composition contains known active agents and that are used normally in the usual hair preparations as the main support, but its main base is made up by the addition of other main products, which are the ones that contribute to the novelty and the inventive action. These products are: spironolactone and flutamide, which can be used on their own or mixing the two of them.

The pharmacological action of these products is as follows:
- The spironolactone acts by reducing the hiperaldosteronism, this means the excess of aldosterone. It is a corticoid mineral with a structure very similar to that of the male hormones that cause alopecia. In the composition that is aim of the invention quantities (weight/final volume) of 0.005-0.5% are used.
- The flutamide acts as a male hormone inhibitor, such as the testosterone and dihydrotestosterone, preventing their penetration into the target cells of the hair follicles and sebaceous glands. In so doing these results in a reduction in the relationship of male/female hormones, bringing about a relative increase of oestrogen that causes the increase of the hair mass and volume. This product, when added to the composition object of the invention is made in quantities (weight/final volume) of 0.02-0.5%.

The two above-mentioned substances, spironolactone and flutamide, either on their own or mixed together bring about an increase in the number, size and volume of the hair. In addition, as the application is topical, and only on the area of hair growth, ensures that the concentration of the main active agents and their effects are localised in this area, which does not occur if it is presented in other forms for use orally or by means other than the gastro-intestinal tract.

Thus, the aim of the invention is to provide a hair composition that encourages the increase and volume of the hair.

Also the aim of the invention is to make a hair composition available that, as well as several other products used conventionally in hair products, contains spironolactone and flutamide as the main active agents, using each one on its own or in combination.

Another aim of the invention is to formulate these main active agents in the form of a hair lotion for topical use on the areas of hair growth, and in so doing avoiding excess concentrations or undesirable side effects on other body organs.

The compositions of the anti-alopecia hair lotions object of this invention are made up from solvents and solubilising agents as a base solution, and also by some main active agents with an action on the hair, several of which are used normally in hair lotions of this type. This is in addition to one or both of the two active agents stated previously as characteristic of this invention.

The basic solutions can have ethyl and/or isopropyl alcohol as the carrier liquid, which can be partially or completely substituted by chloroform, propylene glycol or other polyglycols, with the addition of some quantity of a solubiliser.

The liquid base that contains the spironolactone and the flutamide, used on their own or in combination, also contain the following well known hair substances: Triclosan (bactericide), Octopirox (fungicide), Biotin (vitamin H or hair vitamin), Allantoin (emollient and dermal cell proliferate), Inositol (piloprotelco booster), Panthenol (hair follicle strengthener), lactic acid (to acidify and moisturise the hair growth areas), thyme extract (antiseptic due to the content of thymol and carvacrol), extract of southernwood ("abrotano macho") (hair growth activator), burdock (astringent action and vasoconstrictor, reducer of excess grease in hair growth areas), Panax ginseng extract (with multi-directional hair action), epilobio extract (anti-histamine and anti-inflammatory action) and hydrolysed keratin (carrier of nutrients, amongst which there is cysteine, which is necessary for the existence of hair). The hair regeneration solution can also contain other ingredients such as colourings, perfumes, silicones and derivatives or others.

The procedure for the preparation of the hair regenerator composition of the invention can be summarised as follows:

In a stainless steel, glass or vitro-ceramic container, fitted with an efficient agitation system, a solution is prepared made up of ethanol, isopropanol or polyglycols, with the option of adding solubilisers or not such as polysorbate 80 and others, subsequently adding the active agents of the invention, the spironolactone and the flutamide, either one of them or both mixed together. Subsequently the triclosan, octoprix and biotin are added.

In a similar container as the one mentioned above an aqueous solution is prepared made up of water, in an amount that is sufficient to make the solution up to 100% of the final formula, to which the following are successively added, EDTA ("ethylenediaminetetraacetic acid")(alkaline salt), allentoin, inositol, panthenol and lactic acid.

Next, this second aqueous solution is slowly added, with good agitation, to the first alcohol based solution that contains the main active agents of the invention. Once good dissolution is achieved, the extracts of thyme, southernwood, burdock, ginseng and epilobio, mentioned above, are added. Similarly the hydro-soluble silicone, hydrolysed keratin are added in addition to the colourings or perfumes that are considered appropriate.

The final resulting solution is kept, in suitable containers, at a temperature of 2-5 °C for some five days under an atmosphere of nitrogen. Once this time has elapsed, the solution is filtered through a press filtration process or other suitable system with a micro-pore size, nitrogen is bubbled through the solution, and it is kept in suitable containers that are maintained in a nitrogen atmosphere.

By way of description of the invention, but with the nature of being a limitation, the following examples are stated:

### Example 1.-

In a stainless steel, glass or vitro-ceramic container, fitted with an efficient agitation system, 65 parts of ethyl alcohol are added, 0.005 parts of spironolactone, 0.1 parts of triclosan, 0.08 parts of octopirox and 0.05 parts of biotin, shaking until the complete dissolution is achieved.

In a similar container to the one mentioned above, a sufficient amount of distilled water is added to make up to 100 parts of the final mixture (in this case, some 31 parts of water), and to which the following are successively added, 0.1 parts of EDTA ("ethylenediaminetetraacetic acid") (alkaline salt), 0.1 parts of allentoin, 0.1 parts of inositol, 0.4 parts of panthenol and 0.3 parts lactic acid.

The contents of the second container are slowly poured onto the alcohol solution in the first container. Next the following are added to the mixture, 0.3 parts of thyme extract, 0.3 parts of southernwood extract, 0.2 parts of burdock extract, 0.3 parts of ginseng extract, 0.75 parts of epilobio extract, 0.01 parts of hydro-soluble silicone, 0.03 parts of hydrolysed keratin, and a sufficient amount of a colouring. The mixture is shaken until it is all completely dissolved.

Once the additions have been finished, the contents of the container are transferred to other containers made of stainless steel, glass or vitro-ceramic, which are maintained at a temperature of 2-5 °C for five days in a nitrogen atmosphere. Subsequently the solution is filtered through a press filtration process or other suitable system with a micro-pore size, nitrogen is bubbled through the filtered solution, and it is kept in suitable containers that are kept closed with a nitrogen atmosphere.

### Example 2.-

This is similar to example 1, but the amount of spironolactone is increased up to 0.5% or to amounts in between, or it can be complemented with amounts of flutamide (between 0.02-0.5%) or all of it is substituted by flutamide in the stated amounts.

### Example 3.-

Similar to example 2, but partially or wholly substituting the ethyl alcohol for isopropylic alcohol or by propylene glycol (in an amount greater than 1%) or by mixtures of isopropyl-propylene glycol alcohol, or with the supplementary addition of glycerine, or the partial substitution by chloroform.

### Example 4.-

Similar to any of the previous examples, but partially substituting the ethyl or isopropyl alcohol by polysorbate 80, polysorbate 60, polysorbate 40, polysorbate 20 or Cremophor RH-40.

## Claims

1. Hair regenerator composition, prepared as a lotion, is characterised because in addition to containing a series of known products and product normally used for action on the hair, it contains some as differentiating active ingredients spironolactone and flutamide, it being possible to contain either one or both of these products in sufficient quantities to produce a reduction in the levels of the androgens that cause alopecia, thus, increasing the relative relationship of oestrogen.

2. A composition according to claim 1, characterised because when spironolactone is used as the main active agent, its concentration in the final solution is made up between 0.005-0.5% (weight/volume).

3. A composition according to claim 1, characterised because when flutamide is used as the main active agent, its concentration in the final solution is made up between 0.02-0.5% (weight/volume).

4. A composition according to claims 1-3, characterised because the essential active ingredient can be either one of the two products, spironolactone or flutamide, on their own or mixed together in the stated concentrations.
